# EUROPEAN PATENT SPECIFICATION

(11) **EP 1 131 337 B1**
(45) Date of publication and mention of the grant of the patent: **14.01.2009**
(21) Application number: 99964769.6
(22) Date of filing: 17.11.1999
(51) Int. Cl.: C07J 63/00, A61K 31/56

(54) **NOVEL BETULINIC ACID DERIVATIVES, PROCESSES FOR PREPARING SUCH DERIVATIVES AND ITS USE AS CANCER GROWTH INHIBITORS**
BETULINSÄUREDERIVATE ZUR INHIBIERUNG DES KREBSWACHSTUMS UND EIN VERFAHREN ZU IHRER HERSTELLUNG
DERIVES D'ACIDE BETULINIQUE, PROCEDES DE PREPARATIONS DE TELS DERIVES ET LEURS UTILISATIONS COMME INHIBITEURS DE DEVELOPPEMENT CANCEREUX

(30) Priority: 18.11.1998 IN DE345898
(43) Date of publication of application: 12.09.2001
(73) Proprietor: DABUR PHARMA LTD., Safdarjung Hospital, New Delhi 110 029 (IN)
(72) Inventor: RAMADOSS, Sunder, Haus Khas, New Delhi 110 016 (IN); JAGGI, Mannu, Gurgaon 122 002, Haryana (IN); SIDDIQUI, Mohammad, Jamshed, Ahmed, Ghaziabad 201 002, U.P. (IN); KHANNA, Achla, Behl, Ashok Vihar, New Delhi 110 052 (IN)
(74) Representative: Hucker, Charlotte Jane
(86) International application number: PCT/IN1999/000065
(87) International publication number: WO 2000/046235

(56) References cited:
- EP-A- 0 943 620
- WO-A-98/51293
- WO-A-98/51294
- GB-A- 1 415 601
- CHEMICAL ABSTRACTS, vol. 125, no. 19, 4 November 1996 (1996-11-04) Columbus, Ohio, US; abstract no. 237789, J S LEE ET AL: "cytotoxic constituents from the Forsythiae fructus against L1210 and HL60 cells" page 58; XP002139933 & YAKHAK HOECHI, vol. 40, no. 4, 1996, pages 462-467,
- MILES D H ET AL: "TUMOR INHIBITORS I: PRELIMINARY INVESTIGATION OF ANTITUMOR ACTIVITY OF SARRACENIA FLAVA" JOURNAL OF PHARMACEUTICAL SCIENCES,US,AMERICAN PHARMACEUTICAL ASSOCIATION. WASHINGTON, vol. 63, no. 4, 1 April 1974 (1974-04-01), pages 613-615, XP000611702 ISSN: 0022-3549
- FUJIOKA T ET AL: "ANTI-AIDS AGENTS, 11. BETULINIC ACID AND PLATANIC ACID AS ANTI-HIV PRINCIPLES FROM SYZIGIUM CLAVIFLORUM, AND THE ANTI-HIV ACTIVITY OF STRUCTURALLY RELATED TRITERPENOIDS" JOURNAL OF NATURAL PRODUCTS,XX,XX, vol. 57, no. 2, 1 February 1994 (1994-02-01), pages 243-247, XP000612854 ISSN: 0163-3864
- KIM D S H L ET AL: "Synthesis of betulinic acid derivatives with activity against human melanoma" BIOORGANIC & MEDICINAL CHEMISTRY LETTERS,GB,OXFORD, vol. 8, no. 13, 7 July 1998 (1998-07-07), pages 1707-1712, XP004137114 ISSN: 0960-894X
- KASHIWADA ET AL: "Betulinic Acid and Dihydrobetulinic Acid Derivatives as Potent Anti-HIV Agents" JOURNAL OF MEDICINAL CHEMISTRY., vol. 39, no. 5, 1996, pages 1016-1017, XP002139932 AMERICAN CHEMICAL SOCIETY. WASHINGTON., US ISSN: 0022-2623
- F HASHIMOTO ET AL: "Anti-AIDS Agents. XXVII. Synthesis and Anti-HIV Activity of Betulinic Acid and Dihydrobetulinic Acid Derivatives" BIOORGANIC & MEDICINAL CHEMISTRY,GB,ELSEVIER SCIENCE LTD, vol. 5, no. 12, 1997, pages 2133-2143-2143, XP002102686 ISSN: 0968-0896
- CHEMICAL ABSTRACTS, vol. 35, no. 12, 20 June 1941 (1941-06-20) Columbus, Ohio, US; abstract no. 3997, RIITI KAWAGUTI ET AL: "The constituents of Zyzyphus vulgaris Lamark var. spinosus Bunge" XP002139934 & J. PHARM. CHEM. SOC. JAPAN, vol. 60, 1940, pages 595-596,

## Description

### Field

The invention relates to novel betulinic acid derivatives useful for the inhibition of tumor/cancer cells and a process for preparation of the said derivatives. The invention also provides pharmaceutical compositions useful in the treatment of ovarian, colon, lung, laryngeal, prostate cancer, following on the anti leukemic, and anti-lymphoma activity of said novel betulinic acid derivatives.

### Background and prior art references

Under the auspices of a National Cooperative Natural Product Drug Discovery Group supported by the National Cancer Institute, the potential antitumor activity of approximately 2500 extracts derived from globally collected plants was evaluated in a panel of enzyme based assays and in a battery of cultured human tumor cell lines. One such extract, prepared from the stem bark of *Ziziphus mauritiana* Lam. (Rhamnaceae), displayed selective cytotoxicity against cultured human melanoma cells (Nature Medicine, Vol. 1 (10), 1995, WO 96/29068). As a result of bioactivity guided fractionation, betulinic acid, a pentacyclic triterpene, was identified as a melanoma-specific cytotoxic agent. In follow-up studies conducted with athymic mice carrying human melanomas, tumor growth was completely inhibited without toxicity. As judged by a variety of cellular responses, antitumor activity was mediated by the induction of apoptosis.

A number of triterpenoids, including betulinic acid, have several known medical applications, including use as an anticancer drug. Anderson et al., in WO 95/04526, have discussed the derivatives of triterpenoids which have been used in cancer therapy, including their activity against polyamines which are required by cells to grow at an optimal rate. Some of these triterpenoids have been found to interfere with enzymatic synthesis of polyamines required for optimal cell growth, and thus inhibit the growth of cancer cells, particularly by inhibiting ornithine decarboxylase (Yasukawa, K. et al. Oncology 48 : 72-76,1991). The anti-cancer activity of betulinic acid and some derivatives has been demonstrated using mouse sarcoma 180 cells implanted subcutaneously in nude mice ( JP 87,301,580). Choi et al have shown that betulinic acid 3-monoacetate, and betulinic acid methyl ester exhibit ED₅₀ values of 10.5 and 6.8 µg/ml, respectively, against P388 lymphocytic leukemia cells (Choi, Y-H et al., Planta Medica vol XLVII, pages 511-513, 1988).

Pezzuto et al (US Patent 5,869,535) disclose a method and composition for probes inhibiting tumor growth using betulinic acid or a derivative thereof. Betulinic acid used has been isolated from stem bark of *Ziziphus mauritiana,* by mediating a selective cytotoxic profile against human melanoma in a subject panel of human cancer cell lines, a bioassay directed fractionation based on the profile of bioactivity was conducting using cultured human melanoma cells (MEL-2) as the monitor, and betulinic acid has been obtained therefrom as the active compound. The resulting betulinic acid can be used to inhibit tumor growth, or can be converted to a C-28 betulinic acid derivative to prevent which prevents or inhibits tumor growth. The invention also provides a treatment method using betulinic acid to prevent the growth or spread of cancerous cells, wherein betulinic acid or derivatives thereof is applied in a topical preparation. Betulinic acid was found to inhibit in vitro growth of MEL-2 cells. However, none of the cell lines tested by Pezzuto i.e. [A431 (squamous cells), BC-1 (breast), COL-2 (colon),HT1080 (sarcoma), KB (human oral epidermoid carcinoma), LNCaP (prostate), LU-1 (lung), U373 (glima) and ZR-75-1 (breast) were affected by betulinic acid (ie. ED50 values of greater than 20 µg/ml).

D. S. H. Kim et al. disclose certain oximether derivatives of betulinic acid and the activity against human melanoma (Bioorganic & Medicinal Chemistry Letters 8 (1998), 1701-1712).

Derivatives having ED₅₀ values greater than 4.0 µg/ml are generally considered not to have any significant anticancer activity. Accordingly, the applicants have focussed their research on the possible uses of betulinic acid derivatives against other manifestations of cancer.

### Objects

The main object of the invention is to provide novel betulinic and derivatives, and processes for the preparation thereof, which are useful as anti-cancer drugs.

Another object is to provide pharmaceutical compositions useful for inhibiting and/or preventing growth of cancerous cells, particularly, for inhibiting the .. growth of leukemias and lymphomas and for inhibiting the growth of prostate, larynx ovary, colon and lung cancer using betulinic acid, one or more betulinic acid derivatives or a combination thereof.

Still another object of the invention is to overcome the problem of high toxicity associated with standard chemotherapeutic agents by using a natural product-derived compound, e.g., betulinic acid derivatives.

Yet another object of the invention is to overcome the problem of insufficient availability associated with synthetic anticancer agents using synthetic derivatives of betulinic acid.

### Summary of the invention

The present invention provides a novel betulinic acid derivative as defined in claim 1. The invention also provides pharmaceutical composition comprising said betulinic acid derivative useful for killing or inhibiting the proliferation of cancerous cells. The bio-activity of betulinic acid derivatives is tested using cultured human leukemic cells (MOLT-4), lymphoma cells (U937), prostate cancer cells (DU 145), lung cancer cells (L 132), colon cancer cells (HT29), ovarian cancer cells (PA-1) and laryngeal cancer cells(HeP.2), as the monitor.

The invention also covers use of said novel betulinic acid derivative for the manufacture of a pharmaceutical composition for the treatment for humans, mammals, or other animals suffering from leukemias and lymphomas and in general, in the treatment of prostate cancer, ovarian and lung cancer.

**TABLE I**

| ED₅₀ VALUES (ug/ml) OF BETULINIC ACID DERIVATIVES | | | | | | | | | | | | | |
|---|---|---|---|---|---|---|---|---|---|---|---|---|---|
| | Lymphoma | Deriv | Leukemia | Deriv. | Prostate | Deriv. | Lung | Deriv. | Ovary | Deriv. | Colon | Deriv. | Larynx |
| | 0 937 | | MOLT-4 | | DU145 | | L132 | | PA-1 | | HT-29 | | Hep.2 |
| 1098 | 0.4 | 1098 | 0.5 | 1098 | 1.5 | 1098 | 1.3 | 1098 | 0.9 | 1098 | 2.6 | 1098 | 1.0 |

### In vitro cytotoxic activity of betulinic acid derivatives:

As shown in Table I, in vitro cytotoxic activity of the novel betulinic acid derivative of the present invention (represented by the code number 1098) was determined against human leukemia (MOLT-4), lymphoma cells (U937), prostate cancer cells (DU 145), lung cancer cells (L 132), colon cells (HT29), ovarian cancer cells (PA-1) and laryngeal cancer cells(HeP.2). In vitro cytotoxic activity of novel betulinic acid derivatives was determined by performing the MTT cytotoxicity assay. After 72 hours, the assay was terminated and percent cyotoxicities calculated as shown in Table I.

### Methods of preparation of Betulinic acid derivatives:-

Conventional procedures may be used in the preparation of the betulinic acid derivative of the invention. In the processes herein described, the starting material is betulinic acid or a derivative thereof unless otherwise specifically mentioned. The term "substrate" refers to either betulinic acid, dihydrobetulinic acid or their derivatives with free C₃-hydroxyl and/or C₁₇-carboxylic group used as starting material unless otherwise indicated. Dihydrobetulinic acid is obtained from betulinic acid by reduction of C₂₀₋₂₉. double bond, whereas dihydrobetulinic acid derivatives refers to its derivatisation at either C₂ C₃ and/or C₁₇ positions.

In an embodiment, the process for the preparation of 3-o-benzoyl derivatives of betulinic acid of the present invention comprises the steps of :
i) treating the substrate in organic base with suitable benzoyl chloride derivatives for approximately 6-16 hours at an ambient temperature,
ii) adding water to work up the reaction and extracting with organic solvent to obtain organic layer, and
iii) drying the organic layer over anhydrous sodium sulphate, and evaporating the residue crystallized to yield pure 3-o-benzoyl derivatives.

In this, the organic bases are selected from pyridine and piperidine.

### Pharmaceutical compositions:

In accordance with the practice of the invention, pharmaceutical compositions employing the novel betulinic acid derivative of the invention with pharmaceutically acceptable carriers may be prepared. The pharmaceutical preparations of the invention are synergistic in nature and exhibit surprising properties and effects. The proportion of the active ingredient to the carrier or additives may be in the range of 1:1 to 1:100 preferably, in the range of 1:1 to 1:10.

The pharmaceutical composition prepared may employ betulinic acid derivative of the invention singly or in suitable combinations.

The compositions of this invention may contain a pharmaceutically-acceptable carrier, or physiologically- acceptable diluents, fillers, lubricants, excipents, solvents, binders, stabilizers, and the like. Diluents that may be used in the composition include but or are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the composition include but or are not limited to starch, gelatin and fillers such as sucrose, glucose, dextrose and lactose.

Natural and synthetic gums used in the present invention are selected from sodium alginate, ghatti gum, carboxymethylcellulose methylcellulose, polyvinyl pyrrolidone and veegum. The excipient used the present process is selected from microcystalline cellulose, calcium sulfate, dicalcium phosphate, starch, magnesium stearate, lactose, sucrose. Stabilizers used are polysaccharides such as acacia, agar, alginic acid, guar gum and tragacanth, amphotsics such as gelatin and synthetic and semi-synthetic polymers such as carbomer resins, cellulose ethers, and carboxymethyl chitin.

Solvents that may be used include but are not limited to Ringers solution, water, distilled water, dimethyl, sulfoxide to 50% in water, propylene glycol (near or in water), phosphate buffered saline, balanced salt solution, glycol and other conventional fluids.

The compositions prepared in accordance with the practice of the invention may be administered to subjects in need thereof systemically.

Systemic administration refers to oral, rectal, nasal, transdermal and parental (i.e., intra muscular, intraperitoneal, subcutaneous or intravenous). In accordance with good clinical practice, it is preferred to administer the composition in a dose that will inhibit/prevent growth of cancerous cells without causing undue harmful side effects. The composition may be administered either alone or as a mixture with other therapeutic agents

Compositions which provide from about 10 mg to 1000 mg of the composition per unit dose are preferred. The compositions may be in the form of tablets, lozenges, capsules, powders, aqueous or oily suspensions, syrups, elixirs, implants or aqueous solutions and the like which are prepared by any conventional method. The nature of the composition used will, of course, depend on the desired route of administration. The human dosage of the compounds is in the range of 1.0 to 200 mg/kg/day and the preferred range is 1.0 to 50 mg/kg/day.

### Method of treatment:-

The betulinic acid derivative of the present invention are useful in the treatment a patient with cancer mainly leukemia or lymphoma of prostrate, lung, ovarian, colon or laryngeal cancer, by administering a pharmaceutically effective dosage of betulinic acid derivative or a combination thereof or a formulation containing betulinic acid derivative or a combination thereof to the patient.

### In an embodiment, the subject is a human, mammal or other animal

In another embodiment, the ED₅₀ value of active betulinic acid derivative against leukemia or lymphoma is in the range of 0.34 to 2.00 µg/ml and 0.3 4.00 µg/ml respectively.

In yet another embodiment, the ED₅₀ value of active betulinic acid derivative against prostate cancer is 0.4 to 4.0 µg/ml.

In a further embodiment, ED₅₀ value of the active betulinic acid derivative against lung cancer is in the range of 0.5 to 4.0 µg/ml.

In still another embodiment, value of the active betulinic acid derivative against ovarian cancer is in the range of 0.5 to 4.0 µg/ml.

In still another embodiment, value of the active betulinic acid derivative against colon cancer is in the range of 0.35 to 4.0 µg/ml.

In still another embodiment, value of the active betulinic acid derivative against laryngeal cancer is in the range of 1.0 to 4.0 µg/ml.

In a still further embodiment, the betulinic acid derivative as set out in Table 2 is administered to the subject singly or in combination with pharmaceutically acceptable additives, carriers, diluents, solvents, fillers, lubricants, excipients, binders or stabilizers.

In another embodiment, the pharmaceutical compositions of the invention may be made in various physical forms such as tablets, lozenge, capsule, powder, aqueous or oily suspension, syrup, elixir, implant or aqueous solution.

In one embodiment, the dosage of the composition for humans is in the range of 10 to 200 mg/kg/day.

In another embodiment, the preferred dosage for humans is in the range of 20 to 50 mg/kg/day.

These and other aspects of the invention will become apparent from the specific embodiments and examples described hereinbelow. Various modifications that may be apparent to one in the art are deemed to be encompassed within the scope of the invention.

### Example 1

A suitable formulation of betulinic acid derivative was prepared as follows. The betulinic acid derivative was solubilized in a minimum volume of methanol. The betulinic acid derivative may also be solubilized in isopropyl alcohol, dimethylformamide, dimethylsulfoxide or any other suitable solvent. Substituted beta-cyclodextrin, such as 2-hydroxypropyl beta-cyclodextrin, sulfobutyl ether beta-cyclodextrin was separately dissolved in water to a concentration of approximately 50 to 1000 mg per ml, preferably 250 to 750 mg per ml. The solubilized betulinic acid or its derivative was added in small aliquots to the derivatized beta cyclodextrin solution and sonicated at low temperature until a clear solution developed. The organic solvent was then removed by rotary evaporation and the final solution filtered to give a sterile product. The resulting solution was lyophilized.

### Example 2

In vitro cytotoxic activity of novel betulinic acid derivative was determined by performing the MTT cytotoxicity assay (Mosmann T., J Immunological Methods, 65 : 55 ; 1983). Briefly, the cultured tumor cells were separately seeded in a 96-well culture plate and co-incubated with betulinic acid or its derivatives dissolved in methanol, dimethyl formamide, dimethyl sulfoxide or isopropyl alcohol with relevant controls at 37°C in a CO₂ incubator. After 72 hours, the assay was terminated and percent cyotoxicities calculated. The results are shown in Table I.

## Claims

1. A Betulinic Acid derivative of formula

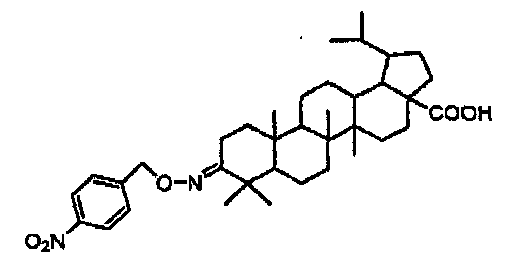

2. A pharmaceutical composition comprising the betulinic acid derivative of claim 1 and a pharmaceutically acceptable carrier or physiologically-acceptable diluent.

3. Use of betulinic acid derivative as claimed in claim 1 for the manufacture of a pharmaceutical composition for the treatment of leukemia or cancer affecting lymphoma, prostate, lungs ovary, colon or larynx.

4. Use as claimed in claim 3, wherein the pharmaceutical composition further comprises a pharmaceutically- acceptable carrier.

5. Use as claimed in claim 3, wherein the pharmaceutical composition is for systemic administration.

6. Use as claimed in claim 3, wherein the cancerous conditions treated are selected from leukemia or cancer afflicting the ovary, colon, lungs, larynx or prostate.

7. Use as claimed in claim 3, for the treatment of an animal.

8. Use as claimed in claim 3, for the treatment of a human.

9. Use as claimed in claim 3, wherein the ED₅₀ value of the betulinic acid derivative against leukemia or lymphoma is in range of 0.34 to 2.00 µg/ml and 0.3 to 4.0 µg/ml respectively.

10. Use as claimed in claim 3, wherein ED₅₀ value of the betulinic acid derivative against prostate cancer is in range of 0.4 to 4.0 µg/ml.

11. Use as claimed in claim 3, wherein ED₅₀ value of the betulinic acid derivative against lung cancer is in range of 0.50 to 4.0 µg/ml.

12. Use as claimed in claim 3, wherein ED₅₀ value of the betulinic acid derivative against ovarian cancer is in range of 0.5 to 4.0 µg/ml.

13. Use as claimed in claim 3, wherein ED₅₀ value of the betulinic acid derivative against colon cancer is in range of 0.35 to 4.0 µg/ml.

14. Use as claimed in claim 3, wherein ED₅₀ value of the betulinic acid derivative against laryngeal cancer is in range of 1.0 to 4.0 µg/ml.

15. Use as claimed in claim 3, wherein the pharmaceutical composition is for administration at a daily dosage in the range of 10 to 200 mg the betulinic acid derivative/ kg patient body weight/ day.

16. Use as claimed in claim 15, wherein the daily dosage is in the range of 20 to 50 mg/kg/day.

## Patentansprüche

1. Betulinsäurederivat der Formel

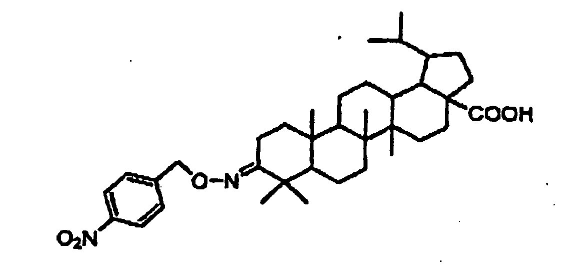

2. Pharmazeutische Zusammensetzung, umfassend das Betulinsäurederivat von Anspruch 1 und einen pharmazeutisch akzeptablen Träger oder ein physiologisch akzeptables Verdünnungsmittel.

3. Verwendung des Betulinsäurederivats wie in Anspruch 1 beansprucht zur Herstellung einer pharmazeutischen Zusammensetzung für die Behandlung von Leukämie oder Krebs, der auf Lymphome, Prostata, Lungen, Eierstöcke, Darm oder Kehlkopf einwirkt.

4. Verwendung wie in Anspruch 3 beansprucht, wobei die pharmazeutische Zusammensetzung ferner einen pharmazeutisch akzeptablen Träger umfasst.

5. Verwendung wie in Anspruch 3 beansprucht, wobei die pharmazeutische Zusammensetzung für die systemische Verabreichung ist.

6. Verwendung wie in Anspruch 3 beansprucht, wobei die behandelten krebsartigen Zustände ausgewählt sind aus Leukämie oder Krebs, der die Eierstöcke, den Darm, die Lungen, den Kehlkopf oder die Prostata befällt.

7. Verwendung wie in Anspruch 3 beansprucht zur Behandlung eines Tieres.

8. Verwendung wie in Anspruch 3 beansprucht zur Behandlung eines Menschen.

9. Verwendung wie in Anspruch 3 beansprucht, wobei der ED₅₀-Wert des Betulinsäurederivats gegen Leukämie oder Lymphome im Bereich von 0,34 bis 2,00 µg/ml bzw. 0,3 bis 4,0 µg/ml liegt.

10. Verwendung wie in Anspruch 3 beansprucht, worin der ED₅₀-Wert des Betulinsäurederivats gegen Prostatakrebs im Bereich von 0,4 bis 4,0 µg/ml liegt.

11. Verwendung wie in Anspruch 3 beansprucht, wobei der ED₅₀-Wert des Betulinsäurederivats gegen Lungenkrebs im Bereich von 0,50 bis 4,0 µg/ml liegt.

12. Verwendung wie in Anspruch 3 beansprucht, worin der ED₅₀-Wert des Betulinsäurederivats gegen Eierstockkrebs im Bereich von 0,5 bis 4,0 µg/ml liegt.

13. Verwendung wie in Anspruch 3 beansprucht, worin der ED₅₀-Wert des Betulinsäurederivats gegen Darmkrebs im Bereich von 0,35 bis 4,0 µg/ml liegt.

14. Verwendung wie in Anspruch 3 beansprucht, wobei der ED₅₀-Wert des Betulinsäurederivats gegen Kehlkopfkrebs im Bereich von 1,0 bis 4,0 µg/ml liegt.

15. Verwendung wie in Anspruch 3 beansprucht, worin die pharmazeutische Zusammensetzung zur Verabreichung mit einer Tagesdosierung im Bereich von 10 bis 200 mg des Betulinsäurederivats/kg Körpergewicht des Patienten/Tag bestimmt ist.

16. Verwendung wie in Anspruch 15 beansprucht, wobei die Tagesdosierung im Bereich von 20 bis 50 mg/kg/Tag liegt.

## Revendications

1. Dérivé d'acide bétulinique de formule

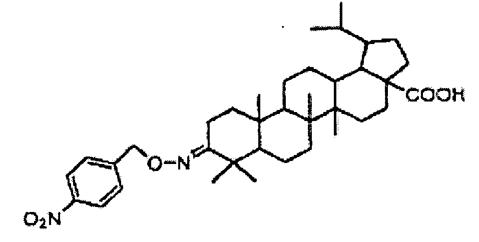

2. Composition pharmaceutique comprenant le dérivé d'acide bétulinique de la revendication 1 et un support pharmaceutiquement acceptable ou un diluant physiologiquement acceptable.

3. Utilisation du dérivé d'acide bétulinique tel que revendiqué dans la revendication 1 pour la fabrication d'une composition pharmaceutique pour le traitement de la leucémie ou d'un cancer affectant un lymphome, la prostate, les poumons, l'ovaire, le colon ou le larynx.

4. Utilisation selon la revendication 3, dans laquelle la composition pharmaceutique qui comprend en outre un support pharmaceutiquement acceptable.

5. Utilisation selon la revendication 3, dans laquelle la composition pharmaceutique est pour à une administration systémique.

6. Utilisation selon la revendication 3, dans laquelle les conditions cancéreuses traitées sont choisies parmi la leucémie ou un cancer affectant l'ovaire, le colon, les poumons, le larynx ou la prostate.

7. Utilisation selon la revendication 3, pour le traitement d'un animal.

8. Utilisation selon la revendication 3, pour le traitement d'un humain.

9. Utilisation selonla revendication 3, dans laquelle la valeur de DE₅₀ du dérivé d'acide bétulinique contre la leucémie ou le lymphome est comprise dans l'intervalle de 0,34 à 2,00 µg/ml et de 0,3 à 4,0 µg/ml, respectivement.

10. Utilisation selon la revendication 3, dans laquelle la valeur de DE₅₀ du dérivé d'acide bétulinique contre le cancer de la prostate est comprise dans l'intervalle de 0,4 à 4,0 µg/ml.

11. Utilisation selon la revendication 3, dans laquelle la valeur de DE₅₀ du dérivé d'acide bétulinique contre le cancer du poumon est comprise dans l'intervalle de 0,50 à 4,0 µg/ml.

12. Utilisation selon la revendication 3, dans laquelle la valeur de DE₅₀ du dérivé d'acide bétulinique contre le cancer de l'ovaire est comprise dans l'intervalle de 0,5 à 4,0 µg/ml.

13. Utilisation selon la revendication 3, dans laquelle la valeur de DE₅₀ du dérivé d'acide bétulinique contre le cancer du colon est comprise dans l'intervalle de 0,35 à 4,0 µg/ml.

14. Utilisation selon la revendication 3, dans laquelle la valeur de DE₅₀ du dérivé d'acide bétulinique contre le cancer du larynx est comprise dans l'intervalle de 1,0 à 4,0 µg/ml.

15. Utilisation selon la revendication 3, dans laquelle la composition pharmaceutique est destinée à être administrée à une dose quotidienne dans l'intervalle de 10 à 200 mg du dérivé d'acide bétulinique/kg de poids corporel du patient/jour.

16. Utilisation selon la revendication 15, dans laquelle la dose quotidienne est comprise dans l'intervalle de 20 à 50 mg/kg/jour.
